# EUROPEAN PATENT SPECIFICATION

(11) **EP 3 233 185 B1**
(45) Date of publication and mention of the grant of the patent: **26.06.2019**
(21) Application number: 15820633.4
(22) Date of filing: 18.12.2015
(51) Int. Cl.: A61N 1/39, A61N 1/04, A61N 1/372, A61B 5/00, A61B 5/1455, A61B 5/0205, A61B 5/024

(54) **WEARABLE CARDIOVERTER DEFIBRILLATOR (WCD) APPARATUS AND METHOD FOR IMPROVED COMFORT AND LONGER WEAR**
AM KÖRPER TRAGBARE KARDIOVERTER-DEFIBRILLATOR-VORRICHTUNG UND VERFAHREN FÜR VERBESSERTEN KOMFORT UND LÄNGERE TRAGEZEIT
APPAREIL DÉFIBRILLATEUR À SYNCHRONISATION AUTOMATIQUE PORTABLE (WCD) ET PROCÉDÉ POUR UN CONFORT AMÉLIORÉ ET UN PORT PLUS LONG

(30) Priority: 18.12.2014 US 201462093495 P
(43) Date of publication of application: 25.10.2017
(73) Proprietor: Koninklijke Philips N.V., 5656 AE Eindhoven (NL)
(72) Inventor: JORGENSON, Dawn Blilie, 5656 AE Eindhoven (NL)
(74) Representative: Müller, Frank
(86) International application number: PCT/IB2015/059764
(87) International publication number: WO 2016/098062

(56) References cited:
- US-A1- 2010 298 899
- US-A1- 2011 288 604
- US-A1- 2012 150 008

## Description

The present embodiments relate generally to wearable cardioverter defibrillator (WCD) apparatus and more particularly, to a WCD apparatus featuring patient health status detection for improved comfort and longer wear, further for potentially easier and more reliable detection of a change in patient condition that may signal or differentiate a need for further analysis (e.g., ECG) and a method thereof. US 2010/298899 A1 discloses a WCD.

At least one known wearable cardioverter defibrillator (WCD) currently on the market uses two sets of electrodes. One set of electrodes comprise dry sensing electrodes for ECG assessment and shock determination. The other set of electrodes is for application of a therapeutic shock. If the sensing electrodes identify a shockable rhythm, the therapeutic electrodes are deployed thru exploding gel. However, with the known WCD, it is well known that the average patient with the WCD only wears it about 5-6 hours a day due to discomfort associated with wearing the known WCD. In particular, patients wearing the known WCD suffer with messy or uncomfortable electrodes on their skin on a continuous basis, which has been a significant compliance problem with existing wearable defibrillators.

Accordingly, an improved method and apparatus for overcoming the problems in the art is desired.

The invention is defined in the independent claims. In the following any aspect, example and embodiment which does not fall under the scope of the independent claims is not part of the invention.

In one embodiment of the present disclosure, a method is disclosed for an improvement to alerting of the WCD system to a potential arrhythmia and for providing more comfortable wear for the patient. The embodiments of the present disclosure also advantageously provide an advancement that improves a wearability of a WCD, which promotes increased wear time of the WCD, and thereby increasing a safety to the patient.

The embodiments of the present disclosure further relate to wearable defibrillators which activate electrode contact in response to non-electrode sensors. The sensors include accelerometers and blood oxygen detectors (e.g. photoplethysmographic detectors). In one embodiment, an advanced Wearable Cardioverter Defibrillator with increased wear time uses non-electrode sensors, in particular, blood oxygen detectors (e.g. photo-plethysmographic detectors) and accelerometer sensors to activate a therapy electrode.

According to one embodiment, a wearable cardioverter defibrillator (WCD) comprises a set of electrodes configured for placement on a subject, the set of electrodes at least operable to sense an ECG signal from the subject. The WCD further comprises a means for electrically engaging the set of electrodes to the subject's skin. At least one non-invasive physiologic sensor is configured for placement on the subject, wherein the at least one non-invasive physiologic sensor comprises one or more of a photoplethysmographic (PPG) sensor and accelerometer sensor. In addition, the WCD comprises a controller configured to monitor an output of the at least one non-invasive physiologic sensor for detecting a change in a health parameter of the subject. The change in health parameter of the subject can be indicative of one or more of a change in subject condition that may be a precursor to potential cardiac arrhythmia or a simultaneously occurring cardiac arrhythmia. In one embodiment, the photoplethysmographic (PPG) sensor is configured to monitor the change in health parameter as a function of arterial oxygen saturation, and the accelerometer sensor is configured to monitor the change in health parameter as a function of respiration and a lack of breathing.

Responsive to detecting the change, the controller activates an alarm for requesting a response from the subject within a predetermined period of time. Responsive to receiving the response from the subject within the predetermined period of time, the controller inhibits the means for electrically engaging the set of electrodes to the subject's skin. Otherwise, responsive to not receiving the response from the subject within the predetermined period of time, the controller initiates the means for electrically engaging the set of electrodes to the subject's skin.

In another embodiment, the set of electrodes comprises a single set of electrodes that is further at least operable to deliver a therapeutic shock to the subject. In this embodiment, the controller is further operable to deliver the therapeutic shock in response to an analysis of an ECG signal obtained after the set of electrodes is electrically engaged. In addition, in one embodiment, the set of electrodes comprises dry therapeutic electrodes with self-deploying gel that automatically deploys the gel prior to shock delivery, the therapeutic electrodes being configured for both obtaining of the ECG signal for assessment and shock determination and delivery of the therapeutic shock.

In a further embodiment, the set of electrodes comprises a first set of electrodes operable to sense the ECG signal from the subject and a second set of electrodes operable to deliver a therapeutic shock to the subject. In this embodiment, the engaging means electrically engages the first set of electrodes and the second set of electrodes to the subject's skin. In addition, the controller is operable to initiate the means for electrically engaging (i) the first set of electrodes and (ii) the second set of electrodes. The controller is further operable to obtain an ECG signal after the first set of electrodes is electrically engaged and deliver the therapeutic shock, in response to an analysis of the ECG signal obtained after the first set of electrodes is electrically engaged, after the second set of electrodes is electrically engaged. In one embodiment, the first set of electrodes comprises dry non-adhesive sensing electrodes and the second set of electrodes comprise dry therapeutic electrodes with self-deploying gel that automatically deploys the gel prior to shock delivery.

In accordance with another embodiment, the means for electrically engaging the set of electrodes includes a mechanism for disposing at least one conductive portion of each electrode of the set of electrodes between a non-conductive contact position and a conductive contact position. Responsive to being in the non-conductive contact position, the at least one electrically conductive portion of each electrode of the set of electrodes does not physically engage the set of electrodes for electrical contact to the subject's skin. In addition, responsive to being in the conductive contact position, the at least one electrically conductive portion of each electrode of the set of electrodes physically engages the set of electrodes for electrical contact to the subject's skin.

In yet another embodiment, the WCD further comprises an alarm module coupled to the controller for providing the alarm as activated by the controller. The alarm includes at least one or more of an audible, tactile, or visible alarm. In addition, the WCD comprises a user interface coupled to the controller for receiving the response from the subject. Furthermore, the WCD comprises a wearable garment; wherein the set of electrodes is disposed on at least one surface of the wearable garment adjacent the subject's skin in response to being worn by the subject. In a still further embodiment, the WCD comprises a means for communicating to a remote device, via at least one or more of wireless and wired communication, an occurrence of a therapeutic shock delivery with the set of electrodes.

According to another embodiment, a method of implementing a wearable cardioverter defibrillator (WCD) comprises configuring a set of electrodes for placement on a subject. The set of electrodes are at least operable to sense an ECG signal from the subject. In addition, the method comprises configuring at least one non-invasive physiologic sensor for placement on the subject, wherein the at least one non-invasive physiologic sensor comprises one or more of a photoplethysmographic (PPG) sensor and accelerometer sensor. The method further comprises monitoring, via a controller, an output of the at least one non-invasive physiologic sensor for detecting a change in a health parameter of the subject being indicative of one or more of a change in subject condition that may be a precursor to potential cardiac arrhythmia or a simultaneously occurring cardiac arrhythmia. The photoplethysmographic (PPG) sensor is configured to monitor the change in health parameter as a function of arterial oxygen saturation. The accelerometer sensor is configured to monitor the change in health parameter as a function of respiration and a lack of breathing.

Responsive to detecting the change, the method includes activating, via the controller, an alarm for requesting a response from the subject within a predetermined period of time, wherein (i) responsive to receiving the response from the subject within the predetermined period of time, inhibiting, via the controller, an electrical engagement of the set of electrodes to the subject's skin, and (ii) responsive to not receiving the response from the subject within the predetermined period of time, initiating, via the controller, a control signal for electrically engaging the set of electrodes, via an electrical engagement mechanism, to the subject's skin.

In one embodiment, the step of configuring the set of electrodes further includes configuring a single set of electrodes for being at least operable to deliver a therapeutic shock to the subject, further comprising delivering the therapeutic shock in response to an analysis of an ECG signal obtained after the set of electrodes is electrically engaged.

In another embodiment of the method, the step of configuring the set of electrodes comprises configuring a first set of electrodes operable to sense the ECG signal from the subject and a second set of electrodes operable to deliver a therapeutic shock to the subject. In addition, the step of electrically engaging comprises electrically engaging the first set of electrodes and the second set of electrodes to the subject's skin. Furthermore, initiating the control signal further comprises for electrically engaging (i) the first set of electrodes and (ii) the second set of electrodes, for obtaining an ECG signal after the first set of electrodes is electrically engaged and for delivering the therapeutic shock, in response to an analysis of the ECG signal obtained after the first set of electrodes is electrically engaged, after the second set of electrodes is electrically engaged. In one embodiment, the first set of electrodes comprises dry non-adhesive sensing electrodes and the second set of electrodes comprises dry therapeutic electrodes with self-deploying gel that automatically deploys the gel prior to shock delivery.

According to yet another embodiment, the method comprises electrically engaging the set of electrodes which includes disposing at least one conductive portion of each electrode of the set of electrodes between a non-conductive contact position and a conductive contact position. Responsive to being in the non-conductive contact position, the at least one electrically conductive portion of each electrode of the set of electrodes does not physically engage the set of electrodes for electrical contact to the subject's skin. In addition, Responsive to being in the conductive contact position, the at least one electrically conductive portion of each electrode of the set of electrodes physically engages the set of electrodes for electrical contact to the subject's skin. In a further embodiment, the set of electrodes comprises dry therapeutic electrodes with self-deploying gel that automatically deploys the gel prior to shock delivery, the therapeutic electrodes being configured for both obtaining of the ECG signal for assessment and shock determination and delivery of the therapeutic shock.

In a yet another embodiment, the method further comprises using an alarm module for providing the alarm as activated, wherein the alarm includes at least one or more of an audible, tactile, or visible alarm. A user interface is provided for receiving the response from the subject. In addition, the method further includes disposing the set of electrodes on at least one surface of a wearable garment adjacent the subject's skin in response to being worn by the subject. Still further, the method includes communicating to a remote device, via at least one or more of wireless and wired communication, an occurrence of a therapeutic shock delivery with the set of electrodes.

Still further advantages and benefits will become apparent to those of ordinary skill in the art upon reading and understanding the following detailed description.

The embodiments of the present disclosure may take form in various components and arrangements of components, and in various steps and arrangements of steps. Accordingly, the drawings are for purposes of illustrating the various embodiments and are not to be construed as limiting the embodiments. In the drawing figures, like reference numerals refer to like elements. In addition, it is to be noted that the figures may not be drawn to scale.
Figures 1A and 1B comprise block diagram views of a wearable cardioverter defibrillator (WCD) according to various embodiments of the present disclosure;
Figure 2 is a perspective image and partial block diagram view of a WCD according to an embodiment of the present disclosure;
Figure 3 is a perspective image view of several components of the set of electrodes in a WCD according to various embodiments of the present disclosure;
Figure 4 is a perspective image view of an electronic control module for the WCD according to an embodiment of the present disclosure;
Figure 5 is a front perspective image view of a WCD with the electronic control module being worn by a subject according to an embodiment of the present disclosure;
Figure 6 is a rear perspective image view of a WCD with the electronic control module being worn by a subject according to an embodiment of the present disclosure; and
Figure 7 is a flow diagram view illustrating a method of implementing a wearable cardioverter defibrillator (WCD) according to an embodiment of the present disclosure.

The embodiments of the present disclosure and the various features and advantageous details thereof are explained more fully with reference to the non-limiting examples that are described and/or illustrated in the drawings and detailed in the following description. It should be noted that the features illustrated in the drawings are not necessarily drawn to scale, and features of one embodiment may be employed with other embodiments as the skilled artisan would recognize, even if not explicitly stated herein. Descriptions of well-known components and processing techniques may be omitted so as to not unnecessarily obscure the embodiments of the present disclosure. The examples used herein are intended merely to facilitate an understanding of ways in which the embodiments of the present may be practiced and to further enable those of skill in the art to practice the same. Accordingly, the examples herein should not be construed as limiting the scope of the embodiments of the present disclosure, which is defined solely by the appended claims and applicable law.

It is understood that the embodiments of the present disclosure are not limited to the particular methodology, protocols, devices, apparatus, materials, applications, etc., described herein, as these may vary. It is also to be understood that the terminology used herein is used for the purpose of describing particular embodiments only, and is not intended to be limiting in scope of the embodiments as claimed. It must be noted that as used herein and in the appended claims, the singular forms "a," "an," and "the" include plural reference unless the context clearly dictates otherwise.

Unless defined otherwise, all technical and scientific terms used herein have the same meanings as commonly understood by one of ordinary skill in the art to which the embodiments of the present disclosure belong. Preferred methods, devices, and materials are described, although any methods and materials similar or equivalent to those described herein can be used in the practice or testing of the embodiments.

The embodiments of the present disclosure relate to non-electrode sensing of a patient parameter that indicates a potential cardiac problem. The sensing initiates the deployment of electrodes for more detailed diagnosis. The benefit of these embodiments is that a patient does not have to suffer with messy or uncomfortable electrodes on their skin on a continuous basis, which has been a significant compliance problem with existing wearable defibrillators.

As will be discussed further herein regarding the WCD of the present disclosure, an initial patient assessment is performed through alternative non-invasive physiologic sensors such as photoplethysmographic (PPG) or accerlerometery. Both PPG and accelerometers can be used to detect significant changes in a patient's state; PPG is well known to detect arterial oxygen saturation and accelerometers have been used to detect respiration. Both are easy to use, inexpensive and non-invasive. Importantly, both can be made to be comfortable to wear for long periods of time. In particular, PPG that uses green light and is less sensitive to noise. A sensor such as these in the WCD would monitor the general health of the patient. If a fatal rhythm were to occur, then the non-invasive sensor would identify this occurrence by a significant change in blood O₂ saturation (PPG) or lack of breathing (accelerometer). If a change in the patient's state was detected, an alarm would alert the patient giving the patient an opportunity to stop further action by the WCD. Lacking a patient response, the therapy electrodes would be applied via an electrically engaging mechanism (e.g., thru exploding gel) and a subsequent analysis of an ECG would take place thru the then electrically engaged electrodes to determine if a shockable rhythm was present. In one embodiment, the need for dry sensing electrodes can be eliminated.

Stated in a different manner, according to another embodiment of the present disclosure, the method includes initially monitoring a vital sign other than, or instead of, an ECG. As a result, the method advantageously provides for enabling a much more comfortable way to monitor a health parameter of a person, e.g., continuously monitor a plethsmography signal (or a respiration rate via an accelerometer, etc.) in a mostly healthy person. The vital sign monitoring doesn't have to be that difficult to do because the WCD will be looking for a healthy signal 99.9% of the time. If the pulse (or respiration or other non-ECG signal) indicates a problem, the WCD garment sounds an alarm to alert the wearer. If the wearer does not respond to the alarm, then gel pads (or other electrical engaging mechanisms) of the wearable garment are activated (i.e., electrically engaged with the user's skin) and only then is the ECG analyzed, whereupon defibrillation proceeds if needed. In addition, in one embodiment, the set of electrodes can include two combined ECG monitoring and defibrillation electrodes, each electrode of the single set being used for both ECG monitoring and defibrillation (i.e., no second set of electrodes). Accordingly, this results in providing a much more comfortable garment.

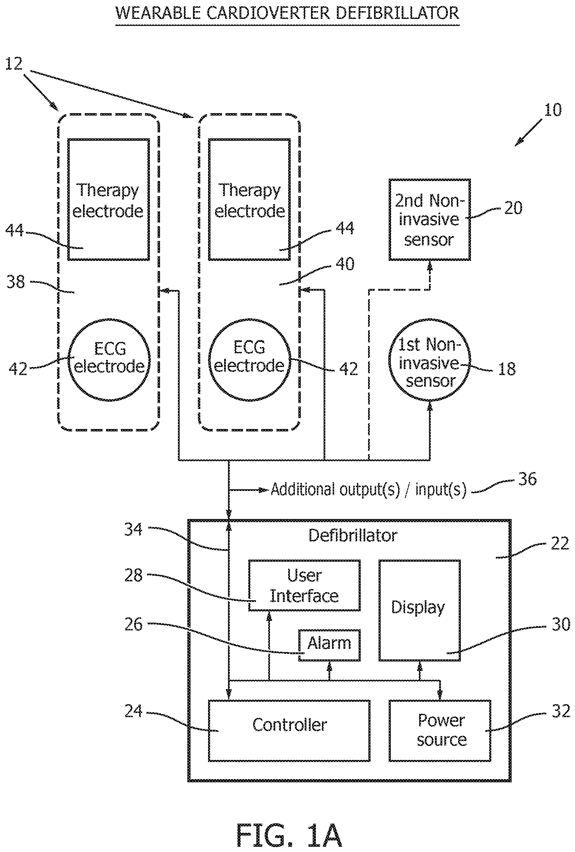

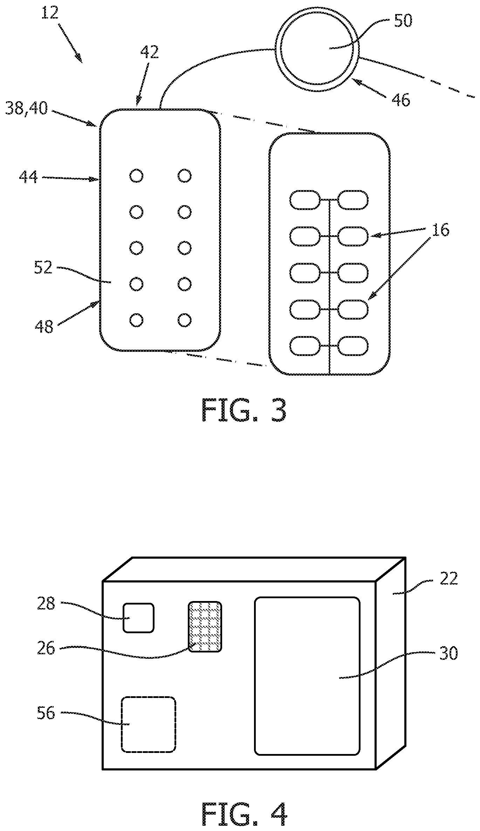

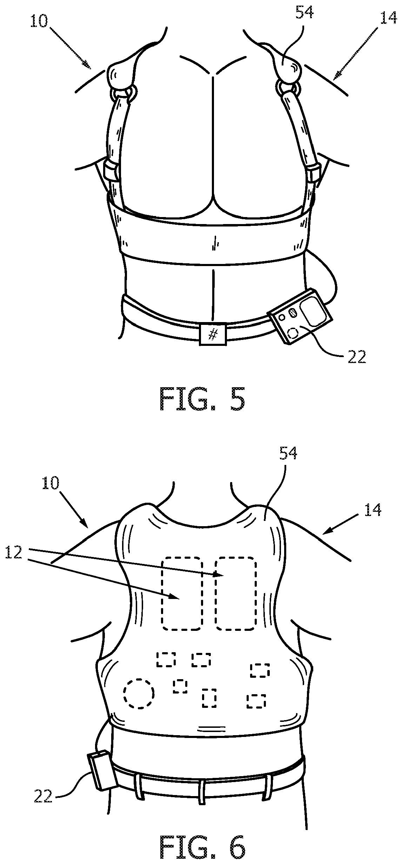

Referring now to Figure 1A, there is shown a block diagram view of a wearable cardioverter defibrillator (WCD) 10 according to one embodiment of the present disclosure. The wearable cardioverter defibrillator 10 includes a set of electrodes 12 configured for placement on a subject 14 (Figures 5 and 6). The set of electrodes 12 is at least operable to sense an ECG signal from the subject. As will be discussed herein with reference to Figure 3, the WCD 10 includes means for electrically engaging 16 the set of electrodes to the subject's skin. The WCD 10 further includes at least one non-invasive physiologic sensor, 18 and/or 20, configured for placement on the subject, wherein the at least one non-invasive physiologic sensor comprises one or more of a photoplethysmographic (PPG) sensor 18 and accelerometer sensor 20.

The WCD 10 further includes a cardioverter defibrillator control module 22 that comprises a controller 24, an alarm 26, a user interface 28, a display 30, and a power source 32. The control module 22 further provides and receives various signals between components of the WCD via signal/power lines, which are generally represented via reference numeral 34. The control module 22 further includes additional output(s) and/or input(s) 36 as may be required for a given wearable cardioverter defibrillator implementation.

The WCD 10 further includes a controller 24 configured to monitor an output of the at least one non-invasive physiologic sensor, 18 and/or 20, for detecting a change in a health parameter of the subject. Controller 24 comprises any suitable processor, microcontroller, or computer for executing the various functions of the embodiments disclosed herein. In particular, one or more output of non-invasive sensors 18 and/or 20 is monitored for detecting a change in health parameter of the subject that is indicative of one or more of a change in subject condition that may be (i) a precursor to potential cardiac arrhythmia or (ii) a simultaneously occurring cardiac arrhythmia. Responsive to detecting the change, the controller 24 activates an alarm 26 for requesting a response from the subject within a predetermined period of time. Subsequent to activating the alarm 26 and responsive to receiving the response from the subject within the predetermined period of time, the controller 24 inhibits an activation of the means for electrically engaging 16 the set of electrodes 12 to the subject's skin. In addition, subsequent to activating the alarm 26 and responsive to not receiving the response from the subject within the predetermined period of time, the controller 24 initiates the means for electrically engaging 16 the set of electrodes 12 to the subject's skin.

In the embodiment illustrated in Figure 1A, the set of electrodes 12 comprises a single set of two electrodes 38 and 40. Each electrode (38,40) includes a combined electrode for being at least operable to sense an ECG signal 42 from the subject and further being at least operable to deliver a therapeutic shock 44 to the subject. In addition, the controller 24 is further operable to deliver the therapeutic shock in response to an analysis of an ECG signal obtained after the set of electrodes is electrically engaged.

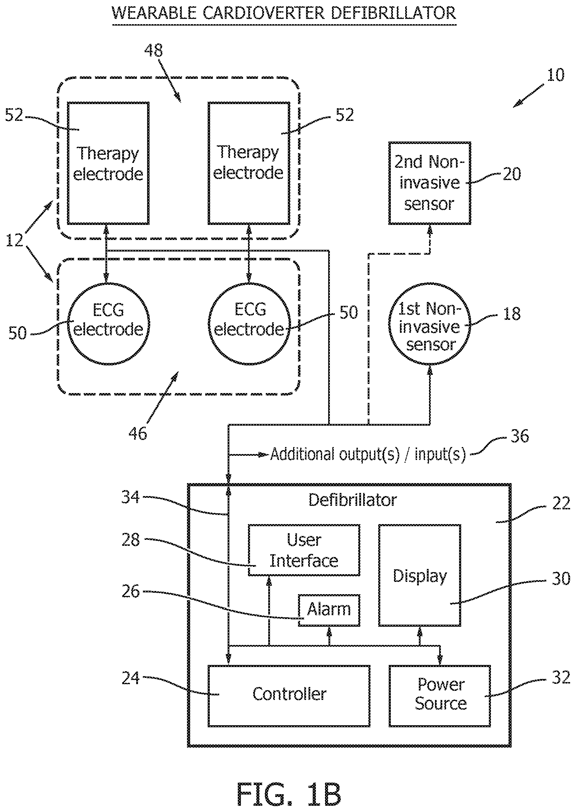

With reference now to Figure 1B, there is shown a block diagram view of a wearable cardioverter defibrillator (WCD) 10 according to another embodiment of the present disclosure. The embodiment of Figure 1B is similar to that of Figure 1A with the following differences. The set of electrodes 12 comprises a first set of electrodes 46 operable to sense the ECG signal from the subject and a second set of electrodes 48 operable to deliver a therapeutic shock to the subject. The first set of electrodes 46 comprises ECG electrodes 50 and the second set of electrodes 48 comprises therapeutic shock electrodes 52. As will be understood further herein, with at least reference to Figure 3, the engaging means 16 electrically engages the first set of electrodes 46 and the second set of electrodes 48 to the subject's skin. In operation, the controller 24 is operable to initiate the means for electrically engaging 16 (i) the first set of electrodes 46 and (ii) the second set of electrodes 48. The controller 24 is further operable to obtain an ECG signal after the first set of electrodes 46 is electrically engaged and deliver the therapeutic shock, in response to an analysis of the ECG signal obtained after the first set of electrodes 46 is electrically engaged, after the second set of electrodes 48 is electrically engaged.

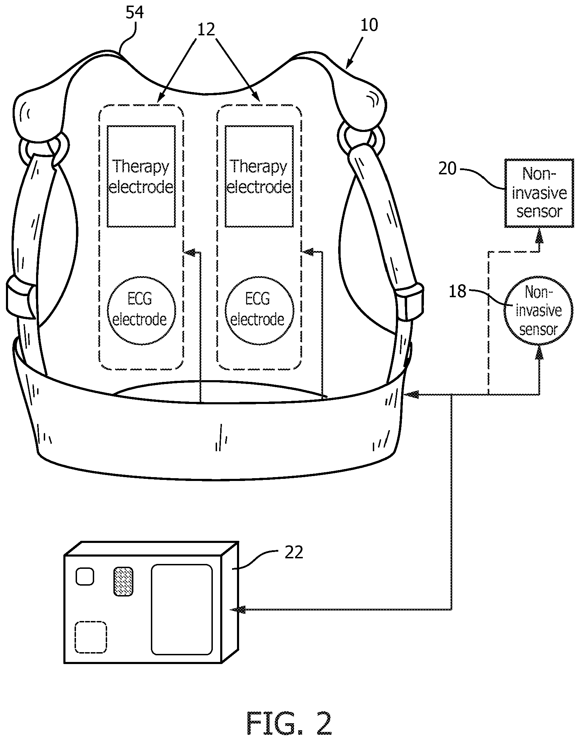

Turning now to Figure 2, there is shown a perspective image and partial block diagram view of a WCD 10 according to an embodiment of the present disclosure. The embodiment of Figure 2 is similar to that of Figures 1A and 1B with the following differences. WCD 10 includes a wearable garment 54 which can comprise any suitable durable fabric and/or material, and of an appropriate size, for being worn comfortably by a subject for extended periods of time (e.g., ∼ 24 hours per day, 7 days per week). The wearable garment 54 should also comprise a washable garment. Accordingly, the wearable garment 54, and thus the WCD 10, is intended to be worn mostly all the time, except when bathing. The set of electrodes 12 is incorporated within the wearable garment 54, and in particular, on an inside portion of the garment. In other words, the set of electrodes is disposed on at least one surface of the wearable garment adjacent the subject's skin in response to being worn by the subject. The set of electrodes 12 are further removable, as needed, e.g., for replacement and/or for washing of the wearable garment 54. When worn by a subject, the set of electrodes 12 is disposed for being adjacent the subject's skin in preparation for use as disclosed herein, further according to the requirements of a given cardioverter defibrillation implementation.

With reference now to Figure 3, a perspective image view of several components of the set of electrodes 12 in the WCD 10 according to various embodiments of the present disclosure is shown. In one embodiment, the set of electrodes 12 comprises a single set of two electrodes 38,40 (only one electrode 38,40 of the set is shown in the figure). Each electrode (38,40) includes a combined electrode for being at least operable to sense an ECG signal 42 from the subject and further being at least operable to deliver a therapeutic shock 44 to the subject. In another embodiment, the set of electrodes 12 comprises a first set of electrodes 46 (only one electrode 50 of the first set is shown in the figure) operable to sense the ECG signal from the subject and a second set of electrodes 48 (only one electrode 52 of the second set is shown in the figure) operable to deliver a therapeutic shock to the subject.

With reference still to Figure 3, the set of electrodes 12 include means for electrically engaging 16 the set of electrodes to the subject's skin. In one embodiment, the means for electrically engaging 16 the set of electrodes 12 comprises a self-deploying gel that automatically deploys the gel in response to an activation signal provided by the controller 24, as discussed herein above. As shown in Figure 3, in one embodiment, the self-deploying gel is configured within a plurality of caps or caplets disposed and arranged for overlying corresponding electrical contact points of a respective electrode. The electrically engaging means 16 thus includes a mechanism for disposing at least one conductive portion of each electrode of the set of electrodes 12 between a non-conductive contact position and a conductive contact position, wherein responsive to being in the non-conductive contact position, the at least one electrically conductive portion of each electrode of the set of electrodes does not physically engage the set of electrodes for electrical contact to the subject's skin, and wherein responsive to being in the conductive contact position, the at least one electrically conductive portion of each electrode of the set of electrodes physically engages the set of electrodes for electrical contact to the subject's skin. Other implementations of a mechanism for electrically engaging the set of electrodes to the subject's skin are contemplated, for example, including a fluid or pressurized bladder arrangement for deploying the electrodes for electrical contact to the subject's skin.

In one embodiment, the set of electrodes 12 comprises dry therapeutic electrodes with self-deploying gel that automatically deploys the gel prior to shock delivery, the therapeutic electrodes being configured for both obtaining of the ECG signal for assessment and shock determination and delivery of the therapeutic shock. In another embodiment, the first set of electrodes 46 comprises dry non-adhesive sensing electrodes and the second set of electrodes 48 comprise dry therapeutic electrodes with self-deploying gel that automatically deploys the gel prior to shock delivery.

With reference now to Figure 4, a perspective image view of an electronic control module 22, also referred to as a cardioverter defibrillator control module, for the WCD 10 according to an embodiment of the present disclosure is shown. The electronic control module 22 is configured for being worn by the subject, for example, being clipped to a belt, supported via a shoulder strap, or other suitable method. Also, with reference to Figures 1A and 1B, the WCD 10 includes an alarm module 26 coupled to the controller 24 for providing the alarm as activated by the controller. The alarm includes at least one or more of an audible, tactile, or visible alarm. In addition, the electronic control module 22 includes a user interface 28 coupled to the controller 24 for receiving the response from the subject. In one embodiment, the user interface 28 comprises a reset button. In another embodiment, the user interface 28 can comprise a touch screen display. Other forms of user interface are also contemplated, such as a voice command interface. Still further, the electronic control module 22 includes a means for communicating 56 to a remote device (not shown), via at least one or more of wireless and wired communication, an occurrence of a therapeutic shock delivery with the set of electrodes. The communicating means 56 (Figure 4) can comprise any suitable transmitter/receiver (Tx/Rx) coupled to controller 24 (Figures 1A and 1B), for implementing a desired communication to/from a remote device (not shown). Such a desired communication may include an emergency type interface or similar for communicating with emergency medical professionals.

Turning now to Figure 5, a front perspective image view of a WCD 10 with the electronic control module 22 being worn by a subject 14 according to an embodiment of the present disclosure is shown. In this illustration, the electronic control module 22 is worn by attaching the module to the subject's belt. Figure 6 shows a rear perspective image view of the WCD 10 with the electronic control module 22 being worn by a subject 14. In addition, the set of electrodes 12 is illustrated via dashed lines, indicative of being on an inside surface of the wearable garment 54 and adjacent to the subject's skin. Other components (e.g., electrodes, wires, sensors, etc.) are also illustrated in dashed lines.

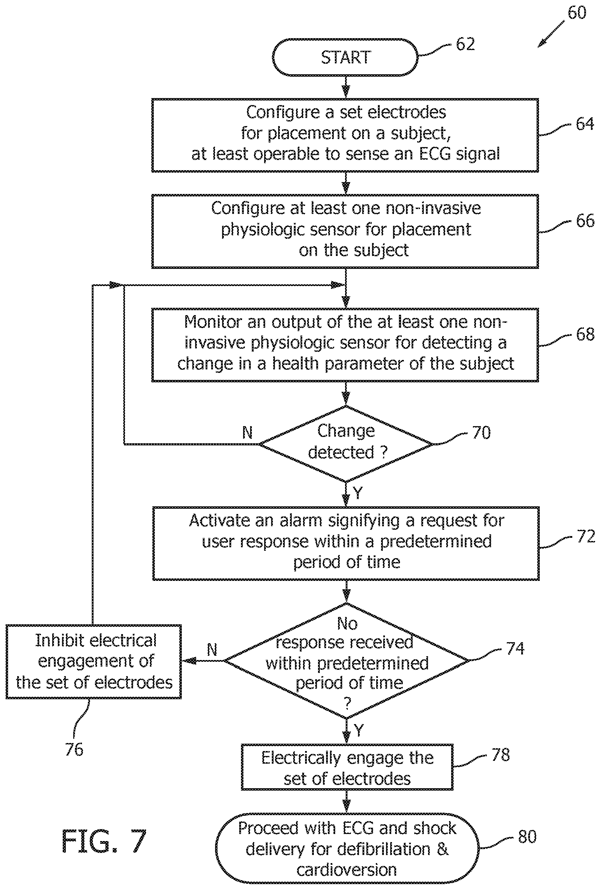

With reference now to Figure 7, a flow diagram view illustrating a method 60 of implementing a wearable cardioverter defibrillator (WCD) according to an embodiment of the present disclosure shall be described. Upon initialization at start (Step 62), the method begins (Step 64) with configuring a set of electrodes for placement on a subject, wherein the set of electrodes is at least operable to sense an ECG signal. The method continues (Step 66) with configuring at least one non-invasive physiologic sensor for placement on the subject, via wearing or other suitable comfortable manner. In a next step, the method includes monitoring an output of the at least one non-invasive physiologic sensor for detecting a change in a health parameter of the subject (Step 68). The method proceeds with a query of whether or not a change been detected (Step 70). If no change has been detected, then the method loops back to monitoring the output of the at least one non-invasive physiologic sensor (Step 68). However, if a change has been detected, the method proceeds by activating an alarm signifying a request for a user response within a predetermined period of time (Step 72). The method proceeds with a query of whether NO user response has been received within the predetermined period of time (Step 74). Responsive to a user response being received within the period of time, the method proceeds by inhibiting an electrical engagement of the set of electrodes (Step 76) and subsequently looping back to monitoring the output of the at least one non-invasive physiologic sensor (Step 68). However, if NO response a change has been detected, the method proceeds with electrically engaging the set of electrodes (Step 78). Subsequent to electrically engaging the set of electrodes, the method proceeds (Step 80) with ECG and shock delivery for defibrillation and cardioversion, as necessary, for the given situation.

Although only a few exemplary embodiments have been described in detail above, those skilled in the art will readily appreciate that many modifications are possible in the exemplary embodiments without materially departing from the novel teachings and advantages of the embodiments of the present disclosure. For example, actual placement of the electrodes and sensors (PPG and accelerometers as well as therapeutic and sensing electrodes) is not limited to that which is shown in the figures and described in the text herein. The embodiments of the present disclosure also cover implementations where the sensors are actually placed in optimal locations for a given WCD application. For example, the PPG/accelerometer sensor may be on the wrist of a patient or worn around the neck (like a pendant) or embedded in the garment or placed in another location. Also, the active (i.e., shocking/theraputic electrodes) need to be in a suitable configuration that covers the heart, e.g., one on the back (posterior) and one on the front (anterior). In one embodiment, one active electrode could be located on a belt portion of a garment that comes around the front of the patient, so that the therapy to the heart is applied between the active electrodes. Other electrode configurations may be possible for use in the WCD. Accordingly, all such modifications are intended to be included within the scope of the embodiments of the present disclosure as defined in the following claims. In the claims, means-plus-function clauses are intended to cover the structures described herein as performing the recited function and not only structural equivalents, but also equivalent structures.

In addition, any reference signs placed in parentheses in one or more claims shall not be construed as limiting the claims. The word "comprising" and "comprises," and the like, does not exclude the presence of elements or steps other than those listed in any claim or the specification as a whole. The singular reference of an element does not exclude the plural references of such elements and vice-versa. One or more of the embodiments may be implemented by means of hardware comprising several distinct elements, and/or by means of a suitably programmed computer. In a device claim enumerating several means, several of these means may be embodied by one and the same item of hardware. The mere fact that certain measures are recited in mutually different dependent claims does not indicate that a combination of these measures cannot be used to an advantage.

## Claims

1. A wearable cardioverter defibrillator (WCD) (10) comprising:
a set of electrodes (12) configured for placement on a subject (14), the set of electrodes (12) at least operable to sense an ECG signal from the subject;
means for electrically engaging (16) the set of electrodes (12) to the subject's skin;
at least one non-invasive physiologic sensor (18,20) configured for placement on the subject, wherein the at least one non-invasive physiologic sensor (18,20) comprises one or more of a photoplethysmographic (PPG) sensor (18) and accelerometer sensor (20); and
a controller (24) configured to monitor an output of said at least one non-invasive physiologic sensor (18,20) for detecting a change in a health parameter of the subject (14) being indicative of one or more of a change in subject condition that may be a precursor to potential cardiac arrhythmia or a simultaneously occurring cardiac arrhythmia, wherein responsive to detecting the change, said controller (24) is adapted to activate an alarm (26) for requesting a response from the subject within a predetermined period of time, wherein (i) responsive to receiving the response from the subject within the predetermined period of time, said controller (24) is adapted to inhibit the means for electrically engaging (16) the set of electrodes (12) to the subject's skin, and (ii) responsive to not receiving the response from the subject within the predetermined period of time, said controller (24) is adapted to initiate the means for electrically engaging (16) the set of electrodes (12) to the subject's skin, wherein the set of electrodes (12) comprises a first set of electrodes (46) operable to sense the ECG signal from the subject and a second set of electrodes (48) operable to deliver a therapeutic shock to the subject,
wherein the engaging means (16) is adapted to electrically engage the first set of electrodes (46) and the second set of electrodes (48) to the subject's skin, and
wherein the controller (24) is operable to initiate the means for electrically engaging (16) (i) the first set of electrodes (46) and (ii) the second set of electrodes (48), the controller (24) further operable to obtain an ECG signal after the first set of electrodes (46) is electrically engaged and deliver the therapeutic shock, in response to an analysis of the ECG signal obtained after the first set of electrodes (46) is electrically engaged, after the second set of electrodes (48) is electrically engaged.

2. The WCD (10) of claim 1, wherein the means for electrically engaging (16) the set of electrodes (12) includes a mechanism for disposing at least one conductive portion of each electrode of the set of electrodes (12) between a non-conductive contact position and a conductive contact position, wherein responsive to being in the non-conductive contact position, the at least one electrically conductive portion of each electrode of the set of electrodes (12) does not physically engage the set of electrodes (12) for electrical contact to the subject's skin, and wherein responsive to being in the conductive contact position, the at least one electrically conductive portion of each electrode of the set of electrodes (12) physically engages the set of electrodes (12) for electrical contact to the subject's skin.

3. The WCD (10) of claim 1, further wherein the first set of electrodes (46) comprises dry non-adhesive sensing electrodes and the second set of electrodes (48) comprise dry therapeutic electrodes with self-deploying gel that automatically deploys the gel prior to shock delivery.

4. The WCD (10) of claim 1, further comprising:
an alarm module (26) coupled to the controller (24) for providing the alarm as activated by the controller, wherein the alarm includes at least one or more of an audible, tactile, or visible alarm; and
a user interface (28) coupled to the controller (24) for receiving the response from the subject.

5. The WCD (10) of claim 1, further comprising:
a wearable garment (54), wherein the set of electrodes (12) is disposed on at least one surface of the wearable garment (54) adjacent the subject's skin in response to being worn by the subject (14).

6. The WCD (10) of claim 1, further comprising:
means for communicating (56) to a remote device, via at least one or more of wireless and wired communication, an occurrence of a therapeutic shock delivery with the set of electrodes.

7. The WCD (10) of claim 1, wherein the photoplethysmographic (PPG) sensor (18) is configured to monitor the change in health parameter as a function of arterial oxygen saturation, and wherein the accelerometer sensor (20) is configured to monitor the change in health parameter as a function of respiration and a lack of breathing.

8. A method (60) of implementing a wearable cardioverter defibrillator (WCD) comprising:
configuring a set of electrodes for placement on a subject, the set of electrodes at least operable to sense an ECG signal from the subject (Step 64);
configuring at least one non-invasive physiologic sensor for placement on the subject, wherein the at least one non-invasive physiologic sensor comprises one or more of a photoplethysmographic (PPG) sensor and accelerometer sensor (Step 66); and
monitoring, via a controller, an output of said at least one non-invasive physiologic sensor for detecting a change in a health parameter (Step 68) of the subject being indicative of one or more of a change in subject condition that may be a precursor to potential cardiac arrhythmia or a simultaneously occurring cardiac arrhythmia, wherein responsive to detecting the change, activating, via the controller, an alarm (Step 72) for requesting a response from the subject within a predetermined period of time, wherein (i) responsive to receiving the response from the subject within the predetermined period of time, inhibiting (Step 76), via the controller, an electrical engagement of the set of electrodes to the subject's skin, and (ii) responsive to not receiving the response from the subject within the predetermined period of time, initiating (Step 78), via the controller, a control signal for electrically engaging the set of electrodes, via an electrical engagement mechanism, to the subject's skin, configuring the set of electrodes (Step 64) comprises configuring a first set of electrodes operable to sense the ECG signal from the subject and a second set of electrodes operable to deliver a therapeutic shock to the subject,
wherein electrically engaging (Step 78) comprises electrically engaging the first set of electrodes and the second set of electrodes to the subject's skin, and
wherein initiating the control signal for electrically engaging the set of electrodes further comprises initiating a control signal for (i) electrically engaging the first set of electrodes for obtaining an ECG signal and (ii) electrically engaging the second set of electrodes for delivering the therapeutic shock, in response to an analysis of the ECG signal obtained after the first set of electrodes is electrically engaged, after the second set of electrodes is electrically engaged.

9. The method (60) of claim 8, wherein electrically engaging (Step 78) the set of electrodes includes disposing at least one conductive portion of each electrode of the set of electrodes between a non-conductive contact position and a conductive contact position, wherein responsive to being in the non-conductive contact position, the at least one electrically conductive portion of each electrode of the set of electrodes does not physically engage the set of electrodes for electrical contact to the subject's skin, and wherein responsive to being in the conductive contact position, the at least one electrically conductive portion of each electrode of the set of electrodes physically engages the set of electrodes for electrical contact to the subject's skin.

10. The method (60) of claim 8, further wherein the first set of electrodes comprises dry non-adhesive sensing electrodes and the second set of electrodes comprises dry therapeutic electrodes with self-deploying gel that automatically deploys the gel prior to shock delivery.

11. The method (60) of claim 8, further comprising:
providing an alarm module for providing the alarm as activated, wherein the alarm includes at least one or more of an audible, tactile, or visible alarm; and
providing a user interface for receiving the response from the subject.

12. The method (60) of claim 8, further comprising:
disposing the set of electrodes on at least one surface of a wearable garment adjacent the subject's skin in response to being worn by the subject.

13. The method (60) of claim 8, further comprising:
communicating to a remote device, via at least one or more of wireless and wired communication, an occurrence of a therapeutic shock delivery with the set of electrodes.

14. The method (60) of claim 8, wherein the photoplethysmographic (PPG) sensor is configured to monitor the change in health parameter as a function of arterial oxygen saturation, and wherein the accelerometer sensor is configured to monitor the change in health parameter as a function of respiration and a lack of breathing.

## Patentansprüche

1. Tragbarer Cardioverter-Defibrillator (WCD) (10), umfassend:
einen Satz Elektroden (12), die zur Platzierung an einem Subjekt (14) konfiguriert sind, wobei der Satz Elektroden (12) mindestens funktionsbereit ist, ein EKG-Signal von dem Subjekt zu messen;
ein Mittel zum elektrischen Anlegen (16) des Satzes Elektroden (12) an die Haut des Subjekts;
mindestens einen nichtinvasiven physiologischen Sensor (18,20), der zur Platzierung an dem Subjekt konfiguriert ist, wobei der mindestens eine nichtinvasive physiologische Sensor (18,20) einen oder mehrere eines photoplethysmographischen (PPG) Sensors (18) und eines Beschleunigungsmesser-Sensors (20) umfasst; und
eine Steuerung (24), die konfiguriert ist, eine Ausgabe des mindestens einen nichtinvasiven physiologischen Sensors (18,20) zum Erfassen einer Veränderung eines Gesundheitsparameters des Subjekts (14), indikativ für eine oder mehrere einer Veränderung eines Subjektzustands, die eine Vorstufe für eine potentielle kardiale Arrhythmie oder eine gleichzeitig auftretende kardiale Arrhythmie sein kann, zu überwachen, wobei die Steuerung (24) angepasst ist, in Reaktion auf das Erfassen der Veränderung einen Alarm (26) zum Anfordern einer Reaktion von dem Subjekt innerhalb eines vorgegebenen Zeitraums zu aktivieren, wobei (i) die Steuerung (24) angepasst ist, in Reaktion auf das Empfangen der Reaktion von dem Subjekt innerhalb des vorgegebenen Zeitraums das Mittel zum elektrischen Anlegen (16) des Satzes Elektroden (12) an die Haut des Subjekts zu inhibieren, und (ii) die Steuerung (24) angepasst ist, in Reaktion auf das Nichtempfangen der Reaktion von dem Subjekt innerhalb des vorgegebenen Zeitraums das Mittel zum elektrischen Anlegen (16) des Satzes Elektroden (12) an die Haut des Subjekts zu initiieren,
wobei der Satz Elektroden (12) einen ersten Satz Elektroden (46), die funktionsbereit sind, das EKG-Signal von dem Subjekt zu messen, und einen zweiten Satz Elektroden (48), die funktionsbereit sind, dem Subjekt einen therapeutischen Schock zu verabreichen, umfasst,
wobei das Anlegemittel (16) angepasst ist, den ersten Satz Elektroden (46) und den zweiten Satz Elektroden (48) an die Haut des Subjekts elektrisch anzulegen, und
wobei die Steuerung (24) funktionsbereit ist, das Mittel zum elektrischen Anlegen (16) (i) des ersten Satzes Elektroden (46) und (ii) des zweiten Satzes Elektroden (48) zu initiieren, wobei die Steuerung (24) weiter funktionsbereit ist, nach dem elektrischen Anlegen des ersten Satzes Elektroden (46) ein EKG-Signal zu erhalten und in Reaktion auf eine Analyse des EKG-Signals, das nach dem elektrischen Anlegen des ersten Satzes Elektroden (46) erhalten worden ist, nach dem elektrischen Anlegen des zweiten Satzes Elektroden (48) den therapeutischen Schock zu verabreichen.

2. WCD (10) nach Anspruch 1, wobei das Mittel zum elektrischen Anlegen (16) des Satzes Elektroden (12) einen Mechanismus zum Anordnen mindestens eines leitfähigen Abschnitts jeder Elektrode des Satzes Elektroden (12) zwischen einer nichtleitenden Kontaktposition und einer leitenden Kontaktposition einschließt, wobei der mindestens eine elektrisch leitende Abschnitt jeder Elektrode des Satzes Elektroden (12) in Reaktion darauf, dass er sich in der nichtleitenden Kontaktposition befindet, den Satz Elektroden (12) für einen elektrischen Kontakt mit der Haut des Subjekts nicht physisch anlegt, und wobei der mindestens eine elektrisch leitende Abschnitt jeder Elektrode des Satzes Elektroden (12) in Reaktion darauf, dass er sich in der leitenden Kontaktposition befindet, den Satz Elektroden (12) für einen elektrischen Kontakt mit der Haut des Subjekts physisch anlegt.

3. WCD (10) nach Anspruch 1, wobei weiter der erste Satz Elektroden (46) trockene nichtklebende Messelektroden umfasst und der zweite Satz Elektroden (48) trockene therapeutische Elektroden mit eigenständiger Gelabgabe, die das Gel vor der Verabreichung des Schocks automatisch abgeben, umfasst.

4. WCD (10) nach Anspruch 1, weiter umfassend:
ein Alarmmodul (26), das mit der Steuerung (24) zum Bereitstellen des Alarms wie durch die Steuerung aktiviert verbunden ist, wobei der Alarm mindestens einen oder mehrere eines hörbaren, fühlbaren oder sichtbaren Alarms einschließt; und
eine Benutzeroberfläche (28), die mit der Steuerung (24) zum Empfangen der Reaktion von dem Subjekt verbunden ist.

5. WCD (10) nach Anspruch 1, weiter umfassend:
ein tragbares Kleidungsstück (54), wobei der Satz Elektroden (12) auf mindestens einer Oberfläche des tragbaren Kleidungsstücks (54) angrenzend an die Haut des Subjekts in Reaktion auf das Getragenwerden von dem Subjekt (14) angeordnet ist.

6. WCD (10) nach Anspruch 1, weiter umfassend:
ein Mittel zum Übermitteln (56) eines Eintretens der Verabreichung eines therapeutischen Schocks mit dem Satz Elektroden an eine ferngesteuerte Vorrichtung mittels mindestens einer oder mehrerer einer drahtlosen und einer drahtgebundenen Übermittlung.

7. WCD (10) nach Anspruch 1, wobei der photoplethysmographische (PPG) Sensor (18) konfiguriert ist, die Veränderung eines Gesundheitsparameters als Funktion der arteriellen Sauerstoffsättigung zu überwachen, und wobei der Beschleunigungsmesser-Sensor (20) konfiguriert ist, die Veränderung eines Gesundheitsparameters als Funktion der Atmung und eines Fehlens der Atmung zu überwachen.

8. Verfahren (60) zum Implementieren eines tragbaren Cardioverter-Defibrillators (WCD), umfassend:
Konfigurieren eines Satzes Elektroden zur Platzierung an einem Subjekt, wobei der Satz Elektroden mindestens funktionsbereit ist, ein EKG-Signal von dem Subjekt zu messen (Schritt 64);
Konfigurieren mindestens eines nichtinvasiven physiologischen Sensors zur Platzierung an dem Subjekt, wobei der mindestens eine nichtinvasive physiologische Sensor einen oder mehrere eines photoplethysmographischen (PPG) Sensors und eines Beschleunigungsmesser-Sensors umfasst (Schritt 66); und
Überwachen, mittels einer Steuerung, einer Ausgabe des mindestens einen nichtinvasiven physiologischen Sensors zum Erfassen einer Veränderung eines Gesundheitsparameters (Schritt 68) des Subjekts, indikativ für eine oder mehrere einer Veränderung eines Subjektzustands, die eine Vorstufe für eine potentielle kardiale Arrhythmie oder eine gleichzeitig auftretende kardiale Arrhythmie sein kann, wobei, über die Steuerung, in Reaktion auf das Erfassen der Änderung ein Alarm aktiviert wird (Schritt 72) zum Anfordern einer Reaktion von dem Subjekt innerhalb eines vorgegebenen Zeitraums, wobei (i), über die Steuerung, in Reaktion auf das Empfangen der Reaktion von dem Subjekt innerhalb eines vorgegebenen Zeitraums ein elektrisches Anlegen des Satzes Elektroden an die Haut des Subjekts inhibiert wird (Schritt 76) und (ii), über die Steuerung, in Reaktion auf das Nichtempfangen der Reaktion von dem Subjekt innerhalb des vorgegebenen Zeitraums ein Steuerungssignal initiiert wird (Schritt 78) zum elektrischen Anlegen des Satzes Elektroden an die Haut des Subjekts mittels eines elektrischen Anlegemechanismus,
Konfigurieren des Satzes Elektroden (Schritt 64), das das Konfigurieren eines ersten Satzes Elektroden, die funktionsbereit sind, das EKG-Signal von dem Subjekt zu messen, und eines zweiten Satzes Elektroden, die funktionsbereit sind, dem Subjekt einen therapeutischen Schock zu verabreichen, umfasst,
wobei das elektrischen Anlegen (Schritt 78) das elektrische Anlegen des ersten Satzes Elektroden und des zweiten Satzes Elektroden an die Haut des Subjekts umfasst und
wobei das Initialisieren des Steuerungssignals zum elektrischen Anlegen des ersten Satzes Elektroden weiter das Initialisieren eines Steuerungssignals (i) zum elektrischen Anlegen des ersten Satzes Elektroden zum Erhalten eines EKG-Signals und (ii) zum elektrischen Anlegen des zweiten Satzes Elektroden zum Verabreichen des therapeutischen Schocks in Reaktion auf eine Analyse des EKG-Signals, das nach dem elektrischen Anlegen des ersten Satzes Elektroden erhalten worden ist, nach dem elektrischen Anlegen des zweiten Satzes Elektroden, umfasst.

9. Verfahren (60) nach Anspruch 8, wobei das elektrische Anlegen (Schritt 78) des Satzes Elektroden das Anordnen mindestens eines leitfähigen Abschnitts jeder Elektrode des Satzes Elektroden zwischen einer nichtleitenden Kontaktposition und einer leitenden Kontaktposition einschließt, wobei der mindestens eine elektrisch leitende Abschnitt jeder Elektrode des Satzes Elektroden in Reaktion darauf, dass er sich in der nichtleitenden Kontaktposition befindet, den Satz Elektroden für einen elektrischen Kontakt mit der Haut des Subjekts nicht physisch anlegt und wobei der mindestens eine elektrisch leitende Abschnitt jeder Elektrode des Satzes Elektroden in Reaktion darauf, dass er sich in der leitenden Kontaktposition befindet, den Satz Elektroden für einen elektrischen Kontakt mit der Haut des Subjekts physisch anlegt.

10. Verfahren (60) nach Anspruch 8, wobei weiter der erste Satz Elektroden trockene nichtklebende Messelektroden umfasst und der zweite Satz Elektroden trockene therapeutische Elektroden mit eigenständiger Gelabgabe, die das Gel vor der Verabreichung des Schocks automatisch abgeben, umfasst.

11. Verfahren (60) nach Anspruch 8, weiter umfassend:
Bereitstellen eines Alarmmoduls zum Bereitstellen des Alarms wie aktiviert, wobei der Alarm mindestens einen oder mehrere eines hörbaren, fühlbaren oder sichtbaren Alarms einschließt; und
Bereitstellen einer Benutzeroberfläche zum Empfangen der Reaktion von dem Subjekt.

12. Verfahren (60) nach Anspruch 8, weiter umfassend:
Anordnen des Satzes Elektroden auf mindestens einer Oberfläche eines tragbaren Kleidungsstücks angrenzend an die Haut des Subjekts in Reaktion auf das Getragenwerden von dem Subjekt.

13. Verfahren (60) nach Anspruch 8, weiter umfassend:
Übermitteln eines Eintretens der Verabreichung eines therapeutischen Schocks mit dem Satz Elektroden an eine ferngesteuerte Vorrichtung mittels mindestens einer oder mehrerer einer drahtlosen und drahtgebundenen Übermittlung.

14. Verfahren (60) nach Anspruch 8,wobei der photoplethysmographische (PPG) Sensor konfiguriert ist, die Veränderung eines Gesundheitsparameters als Funktion der arteriellen Sauerstoffsättigung zu überwachen, und wobei der Beschleunigungsmesser-Sensor konfiguriert ist, die Veränderung eines Gesundheitsparameters als Funktion der Atmung und eines Fehlens der Atmung zu überwachen.

## Revendications

1. Défibrillateur cardiaque portable (WCD) (10) comprenant :
un ensemble d'électrodes (12) configuré pour être placé sur un sujet (14), l'ensemble d'électrodes (12) étant au moins opérationnel pour détecter un signal ECG provenant du sujet ;
des moyens pour mettre en prise électriquement (16) l'ensemble d'électrodes (12) sur la peau du sujet ;
au moins un capteur physiologique non-invasif (18,20) configuré pour être placé sur le sujet, dans lequel le au moins un capteur physiologique non-invasif (18,20) comprend un ou plusieurs capteurs parmi un capteur photopléthysmographique (PPG) (18) et capteur accéléromètre (20) ; et
un dispositif de commande (24) configuré pour surveiller une sortie dudit au moins un capteur physiologique non-invasif (18, 20) pour détecter un changement d'un paramètre de santé du sujet (14) indiquant un ou plusieurs changements d'une condition de sujet qui peut être un précurseur d'arythmie cardiaque potentielle ou une arythmie cardiaque se produisant simultanément, dans lequel, en réponse à une détection du changement, ledit dispositif de commande (24) est adapté pour activer une alarme (26) pour demander une réponse du sujet dans une période de temps prédéterminée, dans lequel (i) en réponse à une réception de la réponse du sujet dans la période de temps prédéterminée, ledit dispositif de commande (24) est adapté pour inhiber les moyens pour mettre en prise électriquement (16) l'ensemble d'électrodes (12) sur la peau du sujet, et (ii) en réponse à la non-réception de la réponse du sujet dans la période de temps prédéterminée, ledit dispositif de commande (24) est adapté pour déclencher les moyens pour mettre en prise électriquement (16) l'ensemble d'électrodes (12) sur la peau du sujet,
dans lequel l'ensemble d'électrodes (12) comprend un premier ensemble d'électrodes (46) opérationnel pour détecter le signal ECG provenant du sujet et un second ensemble d'électrodes (48) opérationnel pour délivrer un choc thérapeutique au sujet,
dans lequel les moyens de mise en prise (16) sont adaptés pour mettre en prise électriquement le premier ensemble d'électrodes (46) et le second ensemble d'électrodes (48) sur la peau du sujet, et
dans lequel le dispositif de commande (24) est opérationnel pour déclencher les moyens pour mettre en prise électriquement (16) (i) le premier ensemble d'électrodes (46) et (ii) le second ensemble d'électrodes (48), le dispositif de commande (24) étant en outre opérationnel pour obtenir un signal ECG après que la mise en prise électrique du premier ensemble d'électrodes (46) et délivrer le choc thérapeutique, en réponse à une analyse du signal ECG obtenu après la mise en prise électrique du premier ensemble d'électrodes (46), après la mise en prise électrique du second ensemble d'électrodes (48).

2. WCD (10) selon la revendication 1, dans lequel les moyens pour mettre en prise électriquement (16) l'ensemble d'électrodes (12) incluent un mécanisme pour disposer au moins une partie conductrice de chaque électrode de l'ensemble d'électrodes (12) entre une position de contact non-conductrice et une position de contact conductrice, dans lequel en réponse au fait d'être dans la position de contact non-conductrice, la au moins une partie électriquement conductrice de chaque électrode de l'ensemble d'électrodes (12) ne vient pas physiquement en prise avec l'ensemble d'électrodes (12) pour un contact électrique avec la peau du sujet, dans lequel en réponse au fait d'être à la position de contact conductrice, la au moins une partie électriquement conductrice de chaque électrode de l'ensemble d'électrodes (12) met physiquement en prise l'ensemble d'électrodes (12) pour un contact électrique avec la peau du sujet.

3. WCD (10) selon la revendication 1, dans lequel en outre le premier ensemble d'électrodes (46) comprend des électrodes de détection non-adhésives sèches et le second ensemble d'électrodes (48) comprend des électrodes thérapeutiques sèches avec un gel à déploiement automatique qui déploient automatiquement le gel avant d'administrer un choc.

4. WCD (10) selon la revendication 1, comprenant en outre :
un module d'alarme (26) couplé au dispositif de commande (24) pour produire l'alarme lorsqu'activé par le dispositif de commande, dans lequel l'alarme inclut au moins une ou plusieurs parmi une alarme sonore, tactile ou visible ; et
une interface utilisateur (28) couplée au dispositif de commande (24) pour recevoir la réponse du sujet.

5. WCD (10) selon la revendication 1, comprenant en outre :
un vêtement pouvant être porté (54), l'ensemble d'électrodes (12) étant disposé sur au moins une surface du vêtement pouvant être porté (54) à proximité adjacente de la peau du sujet en réponse au fait d'être porté par le sujet (14).

6. WCD (10) selon la revendication 1, comprenant en outre :
des moyens pour communiquer (56) à un dispositif à distance, via au moins une ou plusieurs parmi une communication sans fil et filaire, la survenue d'une administration de choc thérapeutique à l'aide de l'ensemble d'électrodes.

7. WCD (10) selon la revendication 1, dans lequel le capteur photopléthysmographique (PPG) (18) est configuré pour surveiller le changement d'un paramètre de santé en fonction d'une saturation artérielle en oxygène, et dans lequel le capteur accéléromètre (20) est configuré pour surveiller le changement de paramètre de santé en fonction d'une respiration et d'un manque de souffle.

8. Procédé (60) de mise en oeuvre d'un défibrillateur cardiaque portable (WCD) comprenant les étapes consistant à :
configurer un ensemble d'électrodes pour une mise en place sur un sujet, l'ensemble d'électrodes étant au moins opérationnel pour détecter un signal ECG provenant du sujet (Étape 64) ;
configurer au moins un capteur physiologique non-invasif pour une mise en place sur le sujet, dans lequel le au moins un capteur physiologique non-invasif comprend un ou plusieurs capteurs parmi un capteur photopléthysmographique (PPG) et un capteur accéléromètre (Étape 66) ; et
surveiller, via un dispositif de commande, une sortie dudit au moins un capteur physiologique non-invasif pour détecter un changement d'un paramètre de santé (étape 68) du sujet indiquant un ou plusieurs changements d'une condition de sujet qui peut être un précurseur d'une arythmie cardiaque potentielle ou une arythmie cardiaque se produisant simultanément, dans lequel, en réponse à une détection du changement, activer, via le dispositif de commande, une alarme (Étape 72) pour demander une réponse du sujet dans une période de temps prédéterminée, dans lequel (i) en réponse à une réception de la réponse du sujet dans la période de temps prédéterminée, inhiber (Étape 76), via le dispositif de commande, une mise en prise électrique de l'ensemble d'électrodes sur la peau du sujet, et (ii) en réponse à la non-réception de la réponse du sujet dans les la période de temps prédéterminée, déclencher (Étape 78), via le dispositif de commande, un signal de commande pour mettre en prise électriquement l'ensemble d'électrodes, via un mécanisme de mise en prise électrique, sur la peau du sujet,
la configuration de l'ensemble d'électrodes (Étape 64) comprend la configuration d'un premier ensemble d'électrodes opérationnel pour détecter le signal ECG provenant du sujet et d'un second ensemble d'électrodes opérationnel pour délivrer un choc thérapeutique au sujet,
dans lequel une mise en prise électrique (Étape 78) comprend une mise en prise électrique du premier ensemble d'électrodes et du second ensemble d'électrodes sur la peau du sujet, et
dans lequel le déclenchement du signal de commande pour mettre en prise électriquement l'ensemble d'électrodes comprend en outre le déclenchement d'un signal de commande pour (i) mettre en prise électriquement le premier ensemble d'électrodes pour obtenir un signal ECG et (ii) mettre en prise électriquement le second ensemble d'électrodes pour délivrer le choc thérapeutique, en réponse à une analyse du signal ECG obtenu après la mise en prise électrique du premier ensemble d'électrodes, après la mise en prise électrique du second ensemble d'électrodes.

9. Procédé (60) selon la revendication 8, dans lequel la mise en prise électrique (Étape 78) de l'ensemble d'électrodes inclut de disposer au moins une partie conductrice de chaque électrode de l'ensemble d'électrodes entre une position de contact non-conductrice et une position de contact conductrice, dans lequel en réponse au fait d'être dans la position de contact non-conductrice, la au moins une partie électriquement conductrice de chaque électrode de l'ensemble d'électrodes ne met pas physiquement en prise l'ensemble d'électrodes pour un contact électrique avec la peau du sujet, et dans lequel en réponse au fait d'être dans la position de contact conductrice, la au moins une partie électriquement conductrice de chaque électrode de l'ensemble d'électrodes met physiquement en prise l'ensemble d'électrodes pour un contact électrique avec la peau du sujet.

10. Procédé (60) selon la revendication 8, dans lequel le premier ensemble d'électrodes comprend des électrodes de détection non-adhésives sèches et le second ensemble d'électrodes comprend des électrodes thérapeutiques sèches avec un gel à déploiement automatique qui déploient automatiquement le gel avant d'administrer un choc.

11. Procédé (60) selon la revendication 8, comprenant en outre :
la fourniture d'un module d'alarme pour fournir l'alarme lorsqu'activé, dans lequel l'alarme inclut au moins une ou plusieurs parmi une alarme sonore, tactile ou visible ; et
la fourniture d'une interface utilisateur pour recevoir la réponse du sujet.

12. Procédé (60) selon la revendication 8, comprenant en outre :
la disposition de l'ensemble d'électrodes sur au moins une surface d'un vêtement pouvant être porté à proximité adjacente de la peau du sujet en réponse au fait d'être porté par le sujet.

13. Procédé (60) selon la revendication 8, comprenant en outre :
la communication à un dispositif à distance, via au moins une ou plusieurs parmi une communication sans fil et filaire, de la survenue d'une administration de choc thérapeutique à l'aide de l'ensemble d'électrodes.

14. Procédé (60) selon la revendication 8, dans lequel le capteur photopléthysmographique (PPG) est configuré pour surveiller le changement d'un paramètre de santé en fonction de la saturation artérielle en oxygène, et dans lequel le capteur accéléromètre est configuré pour surveiller le changement de paramètre de santé en fonction d'une respiration et d'un manque de souffle.
